# FASCICULE DE BREVET EUROPEEN

(11) **EP 1 196 378 B1**
(45) Date de publication et mention de la délivrance du brevet: **20.08.2003**
(21) Numéro de dépôt: 00942170.2
(22) Date de dépôt: 08.06.2000
(51) Int. Cl.: C07C 277/02, C07C 279/02

(54) **BICARBONATE D'AMINOGUANIDINE DE PROPRIETES PARTICULIERES ET SON PROCEDE DE FABRICATION**
AMINOGUANIDIN BICARBONAT MIT BESONDEREN EIGENSCHAFTEN UND VERFAHREN ZU IHRER HERSTELLUNG
AMINOGUANIDINE BICARBONATE WITH PARTICULAR PROPERTIES AND METHOD FOR MAKING SAME

(30) Priorité: 16.07.1999 FR 9909257
(43) Date de publication de la demande: 17.04.2002
(73) Titulaire: Atofina, 92800 Puteaux (FR)
(72) Inventeur: BOSSOUTROT, Jean-Michel, F-69230 Saint-Genis Laval (FR); BOURDAUDUCQ, Paul, F-69630 Chaponost (FR)
(86) Numéro de dépôt international: FR0001579
(87) Numéro de publication internationale: WO01005752

(56) Documents cités:
- DD-A- 249 009
- DATABASE WPI Section Ch, Week 198342 Derwent Publications Ltd., London, GB; Class E16, AN 1983-793499 XP002134581 & SU 981 314 A (MATVEEV L G), 19 décembre 1982 (1982-12-19) cité dans la demande

## Description

La présente invention concerne un procédé de fabrication de bicarbonate d'aminoguanidine. Elle a également pour objet un bicarbonate d'aminoguanidine de propriétés particulières.

La fabrication du bicarbonate d'aminoguanidine (BAG) par réaction d'une solution aqueuse de cyanamide avec de l'hydrazine suivie d'une addition de CO₂ est connue. Comme la mise en contact du cyanamide avec de l'hydrazine en milieu alcalin conduit également à la dimérisation de la cyanamide, il est nécessaire de mettre en oeuvre un gros excès de cyanamide pour atteindre un rendement en bicarbonate d'aminoguanidine convenable.

Ainsi le brevet DD 689 191 nous enseigne d'opérer avec un excès de 100 % de cyanamide (i.e. rapport molaire cyanamide/hydrazine = 2/1) pour obtenir un rendement (ramené à l'hydrazine utilisée) de 80 % en BAG, au bout de 60 heures de réaction. Un rendement en BAG d'environ 90 % peut être atteint au bout de 27 heures de réaction lorsqu'on met en oeuvre des solutions concentrées en cyanamide et hydrazine avec un excès de 100 % de cyanamide (DD 730331).

Le cyanamide étant un produit très coûteux, des tentatives pour réduire cet excès ont fait l'objet de nombreux travaux.

En effet, le brevet SU 981 314 divulgue un rapport molaire, cyanamide/hydrazine compris entre 1,25 et 1,8. Il est indiqué qu'un rendement en BAG (ramené à l'hydrazine) de 95 % est obtenu avec un rapport molaire cyanamide/hydrazine de 1,8. Il nous enseigne également que les rendements chutent à 90 et 85 % pour des rapports molaires de 1,5 et 1,25 respectivement.

La même tendance a été observée par d'autres auteurs. Ainsi, une chute d'environ 12 points en rendement a été enregistrée lorsque le rapport molaire cyanamide/hydrazine passait de 1,2 à 1 (DD 249009).

L'ensemble de la littérature dans ce domaine incite l'homme de métier à travailler avec un excès de cyanamide pour obtenir un rendement élevé en bicarbonate d'aminoguanidine.

La société déposante a mis au point un procédé de fabrication de bicarbonate d'aminoguanidine à partir du cyanamide et d'hydrazine et a observé, de façon surprenante, en opérant avec un léger défaut en cyanamide par rapport à la quantité stoechiométrique, des rendements en BAG aussi élevés, voire supérieurs, à ceux atteints avec des procédés mettant en oeuvre un gros excès en cyanamide.

Selon la présente invention, le procédé consiste à faire réagir une solution aqueuse de cyanamide avec une solution aqueuse d'hydrate d'hydrazine en présence de CO₂ caractérisé en ce que l'on opère avec un léger défaut en cyanamide par rapport à la stoechiométrie.

Le rapport molaire cyanamide/hydrazine mis en jeu est de préférence compris entre 0,80 et 0,99 et avantageusement compris entre 0,85 et 0,95.

Le pH du milieu réactionnel est en général compris entre 6,5 et 8, de préférence compris entre 7 et 7,3. Le pH peut être ajusté par tout moyen convenable et notamment à l'aide du CO₂.

La concentration des solutions aqueuses peut varier dans des larges limites. On préfère le plus souvent utiliser une solution aqueuse de cyanamide de concentration comprise entre 15 et 50 % en poids. La concentration en hydrazine dans la solution aqueuse est avantageusement comprise entre 15 et 64 % en poids.

La température du milieu réactionnel est généralement comprise entre 35 et 70°C. Une température comprise entre 40 et 50°C a conduit à un bicarbonate d'aminoguanidine présentant une structure et des propriétés spécifiques très intéressantes commercialement.

Un mode de réalisation consiste à ajuster à l'aide du CO₂ (gaz carbonique) le pH de la solution d'hydrate d'hydrazine jusqu'à la valeur souhaitée, puis à introduire une solution aqueuse de cyanamide dès que la température de la solution d'hydrazine est portée à environ quelques degrés en dessous de celle choisie pour la réaction.

Le pH du milieu réactionnel est maintenu à l'aide du CO₂ à la valeur souhaitée pendant l'introduction, ou coulée, de la solution de cyanamide et toute la durée de réaction.

Un autre mode de réalisation consiste à ajouter simultanément une solution aqueuse d'hydrate d'hydrazine et du gaz carbonique à une solution aqueuse de cyanamide initialement portée à environ quelques degrés en dessous de celle choisie pour la réaction.

Quel que soit le mode de réalisation, la durée totale de la réaction est en général comprise entre 6 et 15 heures et de préférence comprise entre 7 et 10 heures. La durée de la coulée de cyanamide ou d'hydrate d'hydrazine est en général comprise entre 1 et 3 heures et de préférence voisine de 2 heures.

A l'issue de la réaction, le milieu réactionnel est refroidi jusqu'à la température ambiante et le bicarbonate d'aminoguanidine ainsi obtenu est essoré ou filtré et éventuellement séché.

Avec le procédé selon l'invention, des rendements supérieurs à 90 % et de préférence supérieurs à 95 % sont obtenus et avec une pureté supérieure à 99 %, voire même supérieure à 99,5 %.

La présente invention a également pour objet, un bicarbonate d'aminoguanidine ayant une structure et des propriétés spécifiques particulières. Il est caractérisé par un agglomérat quasi sphérique de cristaux de diamètre moyen compris entre 80 et 500 µm. L'agglomérat a de préférence un diamètre moyen compris entre 100 et 250 µm, le diamètre moyen étant déterminé par la granulométrie laser.

Le bicarbonate d'aminoguanidine selon l'invention présente en outre l'avantage de se séparer facilement du milieu réactionnel par tout moyen connu, par exemple filtration ou essorage et séchage, se distinguant ainsi des cristaux plaquettaires.

### PARTIE EXPERIMENTALE

### Exemple 1

On charge à température ambiante dans un réacteur d'un litre, 110,9 g d'hydrate d'hydrazine à 99,2 % de pureté (2,2 moles) et 300 g d'eau déminéralisée. Le pH de la solution aqueuse est voisin de 11. Puis on fait buller du gaz carbonique dans la solution aqueuse pendant environ 1 heure, ce qui représente 58 g ou 1,3 mole de CO₂, jusqu'à l'obtention d'un pH voisin de 7, tout en portant la température de la solution à environ 40°C.

On coule ensuite pendant environ 2 heures, 171,4 g d'une solution aqueuse de cyanamide à 49 % (2 moles) tout en continuant l'addition de CO₂ de manière à maintenir le pH du milieu réactionnel voisin de 7. Pendant la coulée, la température du milieu est portée à 45°C et le milieu est maintenu à cette température pendant 8 heures avec ajustement du pH à une valeur voisine de 7 par petites additions de CO₂.

La quantité totale de CO₂ ajouté est de 104 g soit 2,36 moles.

A la fin de la réaction, on laisse refroidir le milieu réactionnel jusqu'à la température ambiante puis on filtre et on lave les cristaux de BAG avec 250 ml d'eau et enfin on les sèche sous vide à une température comprise entre 35 et 40°C.

On obtient après séchage 260 g de cristaux de pureté 99,7 %, déterminée par dosage à l'acide perchlorique. Le rendement brut par rapport au cyanamide est de 95,6 %.

Les cristaux obtenus sont sous forme d'agglomérats quasi sphérique (cliché N° 1 par microscopie électronique à balayage).

### Exemple 2

On opère comme décrit à l'exemple 1, sauf que la solution aqueuse d'hydrate d'hydrazine est portée à 55°C au lieu de 40°C et que lors de l'ajout du cyanamide, le milieu réactionnel est porté à 65°C et est maintenu à cette température pendant 4 heures.

On obtient après séchage 261,1 g de cristaux sous forme de plaquettes (cliché N° 2) avec une pureté de 99,6 %. Le rendement brut par rapport au cyanamide est de 96 %.

### Exemple 3

On extrapole l'exemple 1 à l'échelle industrielle, en utilisant un réacteur de 15 m³.

Au bout de 20 minutes d'essorage, les agglomérats contiennent un taux d'humidité de 7 % seulement. A la fin de l'essorage, les agglomérats sont quasi sphériques du type de l'exemple 1 avec une distribution granulométrique resserrée exempte de fines particules, inférieures à 40 µm de diamètre.

### Exemple 4

On reproduit l'exemple 2 à l'échelle industrielle, en utilisant un réacteur de 15 m³.

Au bout de 3 heures d'essorage, les plaquettes contiennent un taux d'humidité de 20 % et à la fin de l'essorage, les plaquettes ont un diamètre moyen de 70 µm avec une distribution granulométrique très étalée avec 20 % de population de particules ayant un diamètre inférieur à 20 µm.

### Exemple 5

On reprend le mode opératoire décrit à l'exemple 1 à l'exception de la durée de coulée de la cyanamide qui est de 5 heures au lieu de 2 heures et la durée de réaction après la coulée qui est réduite de 8 à 5 heures.

Le rendement ainsi que la pureté des cristaux BAG obtenus sont similaires à ceux de l'exemple 1. Par contre, les cristaux sont plutôt sous forme de plaquettes (cliché N° 3) et la durée d'essorage est plus importante.

### Exemple 6

On charge à température ambiante dans un réacteur d'un litre, 171,4 g d'une solution aqueuse de cyanamide à 49 % (2 moles) et 300 g d'eau. Le pH de la solution résultante est voisin de 5. On porte ensuite la solution à 40°C, puis on ajoute en 2 heures simultanément 110,9 g d'hydrate d'hydrazine à 99,2 % (2,2 moles) et 75 g (1,7 moles) de CO₂ pour maintenir le pH à environ 7. Le milieu réactionnel est alors maintenu pendant 8 heures à 45°C avec une petite addition de CO₂ pour ajuster le pH à environ 7. La quantité totale de CO₂ ajoutée est de 94,5 g (2,15 moles).

On laisse ensuite refroidir le milieu réactionnel jusqu'à la température ambiante, puis on filtre et on lave le BAG avec 250 ml d'eau. Enfin on le sèche sous vide à une température comprise entre 35 et 40°C.

Après le séchage, on obtient 259 g d'agglomérats de BAG similaire à l'exemple 1, de pureté 99,6 %.

Le rendement brut en bicarbonate d'aminoguanidine est de 95,2 % par rapport au cyanamide.

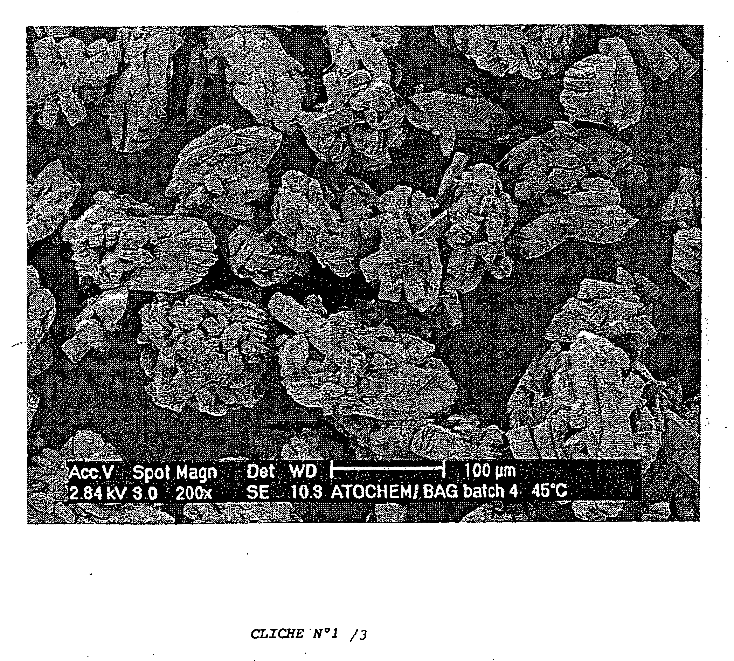

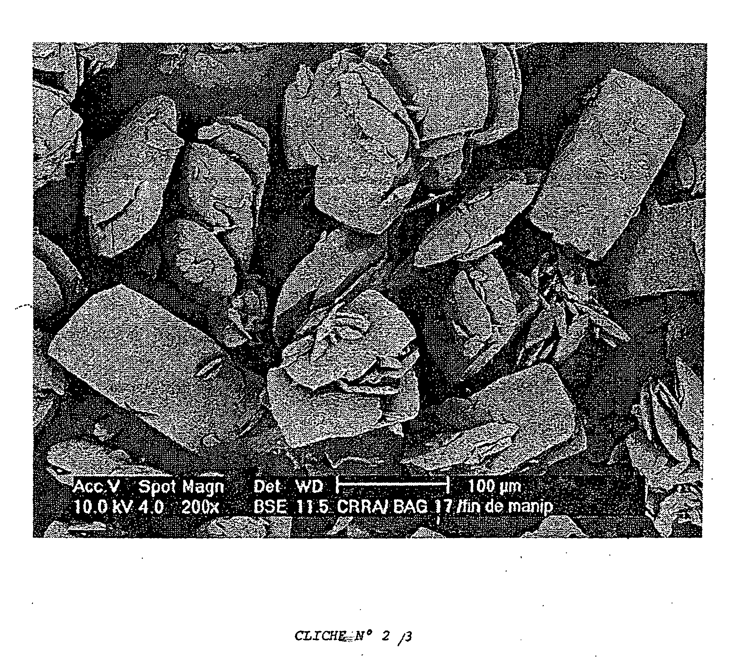

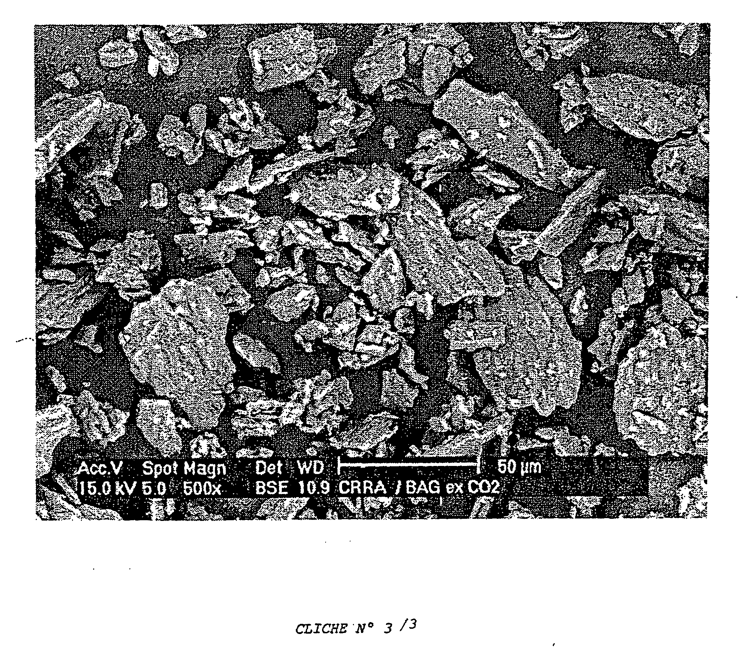

## Revendications

1. Procédé de fabrication de bicarbonate d'aminoguanidine à partir d'une solution aqueuse de cyanamide et d'une solution aqueuse d'hydrate d'hydrazine en présence de CO₂ **caractérisé en ce que** l'on opère avec un léger défaut en cyanamide par rapport à la stoechiométrie.

2. Procédé selon la revendication 1 **caractérisé en ce que** le rapport molaire cyanamide/hydrazine mis en jeu est compris entre 0,8 et 0,99.

3. Procédé selon la revendication 2 **caractérisé en ce que** le rapport molaire cyanamide/hydrazine est compris entre 0,85 et 0,95.

4. Procédé selon l'une des revendications 1 à 3 **caractérisé en ce que** la pH du milieu réactionnel est compris entre 6,5 et 8 et de préférence compris entre 7 et 7,3.

5. Procédé selon l'une des revendications 1 à 4 **caractérisé en ce que** la température du milieu réactionnel est comprise entre 35 et 70°C et de préférence comprise entre 40 et 50°C.

6. Procédé selon l'une des revendications 1 à 5 **caractérisé en ce que** l'on ajuste le pH de la solution d'hydrate d'hydrazine à l'aide du CO₂, puis on introduit la solution aqueuse de cyanamide.

7. Procédé selon l'une des revendications 1 à 5 **caractérisé en ce que** l'on ajoute simultanément une solution aqueuse d'hydrate d'hydrazine et du gaz carbonique à une solution aqueuse de cyanamide.

8. Procédé selon l'une des revendications 6 ou 7 **caractérisé en ce que** la durée de la coulée de cyanamide ou d'hydrate d'hydrazine est comprise entre 1 et 3 heures.

9. Agglomérats quasi sphériques de cristaux de bicarbonate d'aminoguanide de diamètre moyen compris entre 80 et 500 µm et de préférence compris entre 100 et 250 µm.

## Patentansprüche

1. Verfahren zur Herstellung von Aminoguanidinbicarbonat (Aminoguanidinhydrogencarbonat) ausgehend von einer wäßrigen Cyanamidlösung und einer wäßrigen Hydrazinhydratlösung in Gegenwart von CO₂, **dadurch gekennzeichnet, daß** man mit einem leichten Unterschuß an Cyanamid in bezug auf die Stöchiometrie verfährt.

2. Verfahren nach Anspruch 1, **dadurch gekennzeichnet, daß** das eingesetzte Cyanamid/Hydrazin-Molverhältnis zwischen 0,8 und 0,99 liegt.

3. Verfahren nach Anspruch 2, **dadurch gekennzeichnet, daß** das Cyanamid/Hydrazin-Molverhältnis zwischen 0,85 und 0,95 liegt.

4. Verfahren nach Anspruch 1 bis 3, **dadurch gekennzeichnet, daß** der pH-Wert des Reaktionsmilieus zwischen 6,5 und 8, vorzugsweise zwischen 7 und 7,3, liegt.

5. Verfahren nach Anspruch 1 bis 4, **dadurch gekennzeichnet, daß** die Temperatur des Reaktionsmilieus zwischen 35 und 70 °C, vorzugsweise zwischen 40 und 50 °C, liegt.

6. Verfahren nach Anspruch 1 bis 5, **dadurch gekennzeichnet, daß** man den pH-Wert der Hydrazinhydratlösung mit Hilfe von CO₂ einstellt und anschließend die wäßrige Cyanamidlösung hinzugibt.

7. Verfahren nach Anspruch 1 bis 5, **dadurch gekennzeichnet, daß** man gleichzeitig eine wäßrige Hydrazinhydratlösung und Kohlendioxid zu einer wäßrigen Cyanamidlösung hinzugibt.

8. Verfahren nach Anspruch 6 oder 7, **dadurch gekennzeichnet, daß** die Dauer des Zutropfens bzw. der Zugabe des Cyanamids oder Hydrazinhydrats zwischen 1 und 3 Stunden liegt.

9. Quasi-kugelförmige Agglomerate von Aminoguanidinbicarbonatkristallen mit einem mittleren Durchmesser zwischen 80 und 500 µm, vorzugsweise zwischen 100 und 250 µm.

## Claims

1. A process for manufacturing aminoguanidine bicarbonate from an aqueous solution of cyanamide and an aqueous solution of hydrazine hydrate in the presence of CO₂, **characterized in that** the process is performed with a slight deficit of cyanamide relative to the stoichiometry.

2. The process as claimed in claim 1, **characterized in that** the cyanamide/hydrazine molar ratio used is between 0.8 and 0.99.

3. The process as claimed in claim 2, **characterized in that** the cyanamide/hydrazine molar ratio is between 0.85 and 0.95.

4. The process as claimed in one of claims 1 to 3, **characterized in that** the pH of the reaction medium is between 6.5 and 8 and preferably between 7 and 7.3.

5. The process as claimed in one of claims 1 to 4, **characterized in that** the temperature of the reaction medium is between 35°C and 70°C and preferably between 40°C and 50°C.

6. The process as claimed in one of claims 1 to 5, **characterized in that** the pH of the hydrazine hydrate solution is adjusted using CO₂, and the aqueous cyanamide solution is then introduced.

7. The process as claimed in one of claims 1 to 5, **characterized in that** an aqueous hydrazine hydrate solution and carbon dioxide are simultaneously added to an aqueous cyanamide solution.

8. The process as claimed in either of claims 6 and 7, **characterized in that** the duration of the addition of cyanamide or of hydrazine hydrate is between 1 and 3 hours.

9. A virtually spherical aggregate of amino guanidine bicarbonate crystals with a mean diameter of between 80 and 500 µm and preferably between 100 and 250 µm.
